Europäisches Patentamt

European Patent Office

Office européen des brevets

(11) Publication number: 0 368 876 B1

# EUROPEAN PATENT SPECIFICATION

(45) Date of publication of patent specification: **14.09.94**

(51) Int. Cl.5: **A01N 25/06**, A01N 65/00, A61L 9/14, A61L 2/22, F24F 3/12, F24F 3/16

(21) Application number: **88905586.9**

(22) Date of filing: **22.06.88**

(86) International application number:
**PCT/AU88/00199**

(87) International publication number:
**WO 88/10122 (29.12.88 88/28)**

(54) **TREATMENT OF AIR CONDITIONING SYSTEM.**

(30) Priority: **22.06.87 AU 2589/87**

(43) Date of publication of application:
**23.05.90 Bulletin 90/21**

(45) Publication of the grant of the patent:
**14.09.94 Bulletin 94/37**

(84) Designated Contracting States:
**DE FR GB IT NL**

(56) References cited:
**FR-A- 1 588 313          FR-A- 2 414 337
GB-A- 1 554 774          GB-A- 2 160 640
US-A- 3 065 043          US-A- 3 303 091
US-A- 3 493 323**

**JOURNAL OF ECONOMY ENTOMOLOGY, vol. 70, no. 6, 15 December 1977, pages 675-677; B.M. CAWLEY, H.J. RETZER, R.E. MENZER: "performance of three insecticidal aerosols formulated with nonfluorocarbon propellants"**

(73) Proprietor: **THE COMMONWEALTH INDUSTRIAL GASES LIMITED
500 Pacific Highway
St. Leonards, NSW 2065 (AU)**

(72) Inventor: **RYAN, Robert Francis
5 Endeavour Street
Sans Souci, NSW 2219 (AU)**
Inventor: **VALE, Nicholas Frank
50 Osborne Road
Manly, NSW 2095 (AU)**

(74) Representative: **Wickham, Michael et al
c/o Patent and Trademark Department
The BOC Group plc
Chertsey Road
Windlesham Surrey GU20 6HJ (GB)**

**WORLD PATENTS INDEX LATEST, Week 4877, 13 April 1977, Derwent Publications Ltd., London, GB; AN 77-86205Y; & SU-A-552 087**

**CHEMICAL ABSTRACTS, vol. 78, no. 12, 26 March 1973, Columbus, Ohio,US; abstract no. 75772X; J.B. LAWRY: "new constituent of biogenetic, pharmacological and historical interest from melaleuca cajeputi oil"**

**CHEMICAL ABSTRACTS, vol. 82, no. 1, 6 January 1975, Columbus, Ohio, US; abstract no. 672P; D. LAW ET AL.: "antibacterial action of the essential oils of some australian myrtacea..."**

## Description

This invention relates to a method of treating an air conditioning system. The term "air conditioning system" is here used to refer collectively to ducts, fans, filters, humidifiers, coolers and other plant and equipment assembled for air conditioning and, where the context admits, to parts of such systems.

Background Art

In recent years, there has been considerable concern with Legionnaires Disease and with other infections and allergic reactions which can be spread by micro-organisms consisting of bacteria, fungi and protozoans which breed in air conditioning systems under certain conditions. These include Legionella pneumophila and Staphylococcus aureus.

At the present time, the accepted practice for prevention of such diseases is to avoid the accumulation of stagnant water in cooling towers or humidifiers, regularly to clean filters and periodically to treat or scrub filters and air conditioner system surfaces with a chemical disinfectant solution.

The accepted methods suffer from the disadvantages that they are labour intensive and costly to implement. Parts of the system need to be discarded. In many cases ducts cannot be physically reached. The air conditioning system is unusable during the treatment time which may be long. Moreover since the treatment is applicable on a periodic basis, often at intervals weeks or months apart, the risk of infection grows as the length of time since the previous scrubbing increases.

It is known from UK Published Patent Application 2160646 and French Patent 2414337 to provide an air treatment apparatus which includes means for spraying substances, for example, air fresheners or medicaments, into an air stream within an air conditioning unit.

United States Patent 3303091 teaches the use of a composition including oil of Eucalyptus as a decongestant, said composition being suitable for use with pressurised, self-propellant compositions which produce atomised space sprays.

Derwent abstract SU-A-552087 makes reference to a composition for freshening air in enclosed spaces in which the bactericidal and virocidal properties of the composition are improved by the addition of pine oil, lavender oil, linalool, citral and glycerol.

The adaption of the mean droplet size of an aerosol particle for disinfection means is referred to in a document "Theorie und Praxis der Aerosolanwendung in Veterinarmedizin und Tierproduktion" by V S Jarnych.

It is an object of the present invention to provide a method of treating an air conditioning system for preventing the multiplication of pathogenic micro-organisms which thereby avoids or at least ameliorates some of the above discussed disadvantages of previous methods.

Disclosure of the Invention

According to the present invention, a method of treating an air conditioning system comprises the step of admitting to the air conditioning system as a space spray in which the mean droplet size is less than 20 microns, an amount of a biocidal composition comprising an oil of Melaleuca or an active constituent thereof, the biocidal composition being effective against micro-organisms in said system but substantially harmless for humans.

Preferred Embodiments of the Invention

In preferred embodiments the space spray is introduced repetitively at timed intervals into the air conditioning ducting downstream of the heat exchange system.

It will be appreciated that the droplet size indicated is close to a bacterial size and the space spray may be a vapour, fog, aerosol or even a molecular solution of tea tree oil in a carbon dioxide based fluid.

It has not hitherto been practiced to introduce a germicidal, bacteriacidal or fungicidal spray directly and continuously into an air conditioning system for circulation throughout a building. Hitherto, it was considered that such agents were most effective upon contact with bacteria or mould and therefore are best applied directly to contaminated surfaces. Also, it was believed that it would be unacceptable to building occupants to circulate a germicide in the air either because the substances involved might be toxic to humans at an effective concentration or because of the risk of odours or irritation associated therewith. Commonly used germicides such as formaldehyde or carbolic acid are toxic to humans and do not suggest themselves for circulation through a building via an airconditioning system.

3

An embodiment of the invention will now be described by way of example only.

A concentrate comprising Oil of Melaleuca dissolved in alcohol was dissolved in liquid carbon dioxide under pressure. The effective concentration of oil of Melaleuca in the product solution is from 0.1%-2% and preferably approx. 0.3% by weight of solution.

Oil of Melaleuca is an essence produced by the distillation of leaves from the genus Melaleuca in particular the species Melaleuca alternifolia also known as "tea tree" or "ti-tree". It is known to contain mono- and sesquiterpenes including pinenes, terpineols and cineole.

The oil of Melaleuca concentrate possessed the following characteristics:

| Cineole content | 7.0% av. |
|---|---|
| Terpinen-4-ol content | 63.0% min. |
| Specific Gravity @ 20°C | 0.9302 |
| Refractive Index @ 20°C | 1.4763 |

A small quantity of a deodorizer, for example Lauryl Methacrylate, was added to counter the "swampy" smell of oil of Melaleuca and a solvent added to enhance the mutual solubility of the solution components.

The concentrate was admitted to the duct of an operating building air conditioning system downstream of the heat exchange system, as a space spray in which the particles were of from 2 to 20 microns in mean diameter. The building was situated in a tropical climate and had a problem with tendency for mould growth on interior walls. It was found that rooms in the building through which the treated air circulated remained mould-free, while a mould was found to grow on walls in rooms used as a control in which the circulating air was not treated with the spray. The air in the treated rooms was not noticably different in smell from the untreated air and did not cause irritation or discomfort to the occupants of the building.

A bacterial count conducted on surfaces exposed to the treated and untreated control circulating air showed a significant reduction in the number of microorganisms in rooms through which treated air circulated.

A series of tests were performed in which the oil of Melaleuca concentrate was tested according to the procedures of the Therapeutic Goods Act Disinfectant Test, Option A - Hospital Grade Clean. This test is applicable to a surface spray.

The solution was used undiluted. The following results were obtained and show that the solution was effective as a surface spray against a range of microorganisms.

## TABLE 1

Dilution used:  used undiluted

| | Recovery Broth +/- Growth | | Initial Inoculum orgs/ml. | Status |
|---|---|---|---|---|
| | Challenge 1 | Challenge 2 | | |
| **Day 1** | | | | |
| <u>S. aureus</u> | - - - - - | - - - - - | $5.3 \times 10^8$ | Pass |
| <u>E. coli</u> | - - - - - | - - - - - | $6.1 \times 10^8$ | Pass |
| <u>P. aeruginosa</u> | - - - - - | - - - - - | $6.9 \times 10^8$ | Pass |
| <u>P. vulgaris</u> | - - - - - | - - - - - | $8.4 \times 10^8$ | Pass |
| Controls:  all valid | | | | |
| **Day 2** | | | | |
| <u>S. aureus</u> | - - - - - | - - - - - | $2.1 \times 10^8$ | Pass |
| <u>E. coli</u> | - - - - - | - - - - - | $1.1 \times 10^8$ | Pass |
| <u>P. aeruginosa</u> | - - - - - | - - - - - | $8.0 \times 10^8$ | Pass |
| <u>P. vulgaris</u> | - - - - - | - - - - - | $9.5. \times 10^8$ | Pass |
| Controls:  all valid | | | | |

EP 0 368 876 B1

**TABLE 1 (Cont'd.)**

Dilution used: used undiluted

| Day 3 | Recovery Broth +/- Growth | Initial Inoculum orgs/ml. | Status |
|---|---|---|---|
| S. aureus | – – – – – | $3.9 \times 10^8$ | Pass |
| E. coli | – – – – – | $4.0 \times 10^8$ | Pass |
| P. aeruginosa | – – – – – | $3.8 \times 10^8$ | Pass |
| P. vulgaris | – – – – – | $1.1 \times 10^9$ | Pass |

Controls: all valid

A further series of tests was performed wherein surface plated cultures of various microorganisms were exposed to a known concentration of the concentrate injected into a space as a spray having average particle diameter of 2-20 microns over a set period of time and then examined for viability.

More particularly, fresh cultures of test organisms identified as 1 to 4 in Table 2 were prepared from stock culture slopes and sub-cultured into Association of Official Analytical Chemists (AOAC) broth daily for 5 days prior to testing, the fifth day culture was the initial inoculum.

The exception to this procedure was Legionella pneumophila SG1 (identified as 5 in Table 2) which was subcultured onto a BCYE media and then the slope washed with 0.9% Saline solution to give the initial inoculum.

The organisms were diluted to an approximate concentration of $10^1$ and $10^2$ and surface spread onto either Tryptone Soya agar (Organisms 1-4) or a BCYE agar and allowed to air dry for 20 mins.

The inoculated plates were positioned, uncovered, within the test chamber and the sample was introduced into the chamber via a 0.004" diameter crevice nozzle over a 5 second time period.

This equates to a final concentration of sample of 0.43 g/l within the test chamber.

The sample was reintroduced into the cabinet each 24 hrs., as detailed above, and the plates were examined for viability at the exposure times detailed in the results section.

Viability was confirmed by subculturing onto appropriate media.

## TABLE 2

Organisms           (Same for all test procedures)

| | |
|---|---|
| 1 | Staphylococcus aureus NCTC 4163 |
| 2 | Escherischia coli NCTC 8196 |
| 3 | Pseudomonas aeruginosa NCTC 6749 |
| 4 | Proteus vulgaris NCTC 4635 |
| 5 | Legionella Pneumophila SG1 EML 80 |

Series 1          Organisms concentration 10 cfu/plate

| Organism | Time 0 | 6hrs | 1 day | 2 days | 7 days |
|---|---|---|---|---|---|
| 1 | + | + | + | + | − |
| 2 | + | + | + | + | − |
| 3 | + | + | + | + | + |
| 4 | + | + | + | + | − |
| 5 | + | + | + | + | − |

Series 2          Organism concentration 100 cfu/plate

| Organism | Time 0 | 6hrs | 1 day | 2 days | 7 days |
|---|---|---|---|---|---|
| 1 | + | + | + | + | − |
| 2 | + | + | + | + | − |
| 3 | + | + | + | + | + |
| 4 | + | + | + | + | − |
| 5 | + | + | + | + | − |

Controls    Both organism concentrations

Method    As test method but with $CO_2$ instead of test sample

| Organism | Time 0 | 6hrs | 1 day | 2 days | 7 days |
|---|---|---|---|---|---|
| 1 | + | + | + | + | + |
| 2 | + | + | + | + | + |
| 3 | + | + | + | + | + |
| 4 | + | + | + | + | + |
| 5 | + | + | + | + | + |

The results shown in table 2 indicate that the Oil of Melaleuca was effective over 7 days against Staphylococcus aureus and Legionella pneumophila as well as E. coli and Proteus vulgaris when admitted as a space spray.

In further tests concentrates according to the invention were introduced as a fog of particle size less than 20 microns into an air conditioning system for short periods repetitively.

Plates were deposited in the ductwork and microorganism counts at various points in the system were recorded. These further tests confirmed the effectiveness of the treatment.

Oil of Melaleuca contains sesquiterpenes and is a "swampy" smelling oil. It has been used as a mild antiseptic, germicide, bacteriacide and fungicide for which purpose it has been applied as a solution to infected surfaces. It has not previously been known to be effective as a space spray and has not been known to be effective against Legionella Sp.

It is not clear whether the effectiveness of the system is due to a synergism between various oil of Melaleuca components, or to a particular component of oil of Melaleuca, or to synergism between one or more oil of Melaleuca components with the carbon dioxide or the deodorizer or the alcohol or the combination thereof. Any and all such combinations are deemed to be within the scope hereof.

For what ever reason it appears that the treatment is effective in reducing or preventing the growth of Legionella bacteria and that the treatment will also reduce or preent the growth of prevent the growth of staphylococcus and/or other micro-organisms and moulds.

The composition may be admitted to the ducts of an operating air conditioning system under automatic control to maintain a predetermined concentration in the atmosphere or may be injected repetitively at predetermined intervals, for example, half hourly, to produce a desired peak concentration. In a typical system injections of duration 10-30 seconds at 30 minute intervals may be applied throughout the day while the building is occupied and treatment of different duration might be applied before start up or overnight in timer controlled systems.

The treatment need not be applied to a system while in operation. The liquid carbon dioxide based product typically has a vapour pressure of around 5000 kPa at 15°C. When injected into a air conditioning system through a nozzle orifice of from 10-30 thousandths of an inch diameter, the particles have a high velocity and surprisingly reach into parts of the duct which are occluded from the nozzle. Accordingly, the space spray may be effective in an air conditioning system which is closed down.

## Claims

1. A method of treating an air conditioning system comprising the step of admitting to the air conditioning system as a space spray in which the mean droplet size is less than 20 microns, an amount of a biocidal composition comprising an oil of Melaleuca or an active constituent thereof, the biocidal composition being effective against micro-organisms in said system and substantially harmless for humans.

2. A method as claimed in claim 1, in which the mean droplet size is from 2 to 20 microns.

3. A method as claimed in claim 1 or 2, in which the space spray is formed from a solution of the biocidal composition in liquid carbon dioxide.

4. A method as claimed in claim 3, in which the concentration of oil of Melaleuca is from 0.1 to 2.% by weight of the solution.

5. A method according to any one of claims 1 to 4, in which the space spray is admitted to the air conditioning system discontinuously and repetitively.

## Patentansprüche

1. Verfahren zur Behandlung eines Klimaanlagensystems, umfassend die Stufe des Einlassens in das Klimaanlagensystem als Raumspray, in welchem die mittlere Tröpfchengröße weniger als 20 $\mu$m beträgt, einer Menge einer bioziden Zusammensetzung, die ein Öl von Melaleuca oder einen aktiven Bestandteil hiervon umfaßt, wobei die biozide Zusammensetzung gegen Mikroorganismen in diesem System wirksam und praktisch unschädlich für Menschen ist.

2. Verfahren nach Anspruch 1, bei welchem die mittlere Tröpfchengröße von 2 bis 20 $\mu$m beträgt.

**3.** Verfahren nach Anspruch 1 oder 2, bei welchem der Raumspray aus einer Lösung der bioziden Zusammensetzung in flüssigem Kohlendioxid gebildet wird.

**4.** Verfahren nach Anspruch 3, bei welchem die Konzentration von Öl von Melaleuca von 0,1 bis 2 Gew.-% der Lösung beträgt.

**5.** Verfahren nach einem der Ansprüche 1 bis 4, bei welchem der Raumspray in das Klimaanlagensystem diskontinuierlich und wiederholt eingelassen wird.

**Revendications**

**1.** Procédé de traitement épuratoire d'un système de climatisation comprenant l'étape consistant à admettre dans le système de climatisation, sous forme d'une pulvérisation pour locaux dans laquelle la taille moyenne des gouttelettes est inférieure à 20 microns, une quantité d'une composition biocide renfermant une huile de *Melaleuca* ou un constituant actif de celle-ci, la composition biocide étant efficace dans ledit système contre les microorganismes mais réellement sans danger pour l'être humain.

**2.** Procédé selon la Revendication 1, dans lequel la taille moyenne des gouttelettes est comprise entre 2 et 20 microns.

**3.** Procédé selon la Revendication 1 ou 2, dans lequel la pulvérisation pour locaux est produite à partir d'une solution de la composition biocide dans du dioxyde de carbone liquéfié.

**4.** Procédé selon la Revendication 3, dans lequel la concentration de l'huile de *Melaleuca* est comprise entre 0,1 % et 2% en poids de la solution.

**5.** Procédé selon l'une quelconque des Revendications 1 à 4, dans lequel la pulvérisation pour locaux est admise dans le système de climatisation de manière discontinue et répétitive.